# EUROPEAN PATENT SPECIFICATION

(11) **EP 0 370 555 B1**
(45) Date of publication and mention of the grant of the patent: **07.09.1994**
(21) Application number: 89202851.5
(22) Date of filing: 08.11.1989
(51) Int. Cl.: C07C 37/70, C07C 65/05, C07C 51/42

(54) **A process for the separation of a phenol**
Verfahren zur Abtrennung von Phenol
Procédé de séparation d'un phénol

(30) Priority: 23.11.1988 GB 8827306
(43) Date of publication of application: 30.05.1990
(73) Proprietor: SHELL INTERNATIONALE RESEARCH MAATSCHAPPIJ B.V., 2596 HR Den Haag (NL)
(72) Inventor: Bitter, Johan George Albert, NL-1031 CM Amsterdam (NL); Den Boestert, Johannes Leendert Willem Cornelis, NL-1031 CM Amsterdam (NL); Weeres, Wilhelmus Jacob Maria, NL-2596 HR Den-Haag (NL)

(56) References cited:
- DE-C- 466 363
- GB-A- 1 345 276
- GB-A- 1 480 018
- US-A- 3 956 112

## Description

The invention relates to a process for the separation of a phenol from a mixture obtained by carboxylation of the phenol.

When alkyl substituted phenols are converted into alkylsalicylates, the yield is generally about 75%. When recycling to the reactor is applied, the yield is increased to about 82%. Since the alkyl substituted phenols and the alkylsalicylates are difficult to separate in practice until now the phenols have not been removed. In the production of overbased calcium salts of the above-mentioned alkylsalicylic acids, the phenols do no harm. It would, however, be preferable when the alkyl substituted phenols could be separated and used again as starting materials in the preparation of alkylsalicylates. In the overall reaction much higher yields of alkylsalicylates could be obtained, calculated on the alkyl phenols.

British Patent 1,480,018 discloses a process for separating a phenolic material from an aqueous mixture such as waste water, by a non-porous organic polymeric membrane under conditions such that the phenolic material has a lower chemical potential at the permeate side of the membrane. In order to maintain this lower chemical potential a solvent or a complexing agent for the phenol is contacted on the permeate side of the membrane.

Applicant has now found that the phenols can be separated from their mixture with alkyl salicylates in an organic solvent, by the use of a membrane which selectively absorbs the phenols, solubilises them in the membrane matrix, and allows their diffusion through its wall and desorption from its downstream side.

The invention relates to a process for the separation of an alkylphenol, from a mixture obtained by the phenation and carboxylation of an alkylphenol having from 2 to 20 carbon atoms in the alkyl group, the mixture containing the alkylphenol and the corresponding alkylphenolate and alkylsalicylate, in an aromatic hydrocarbon solvent, characterized in that the separation is carried out by maintaining a positive pressure- and concentration gradient across a dense membrane which is selectively permeable to the alkylphenol and which swells by the aromatic hydrocarbon solvent.

The alkyl salicylates can be prepared by the following sequence of reactions:
1. alkylation 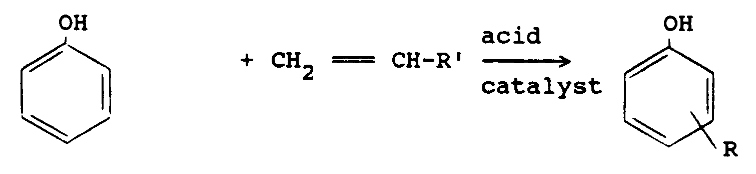 wherein R' is an alkyl group, having 2 C-atoms less than R.
2. phenation 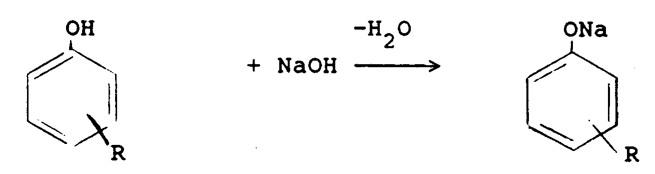
3. carboxylation 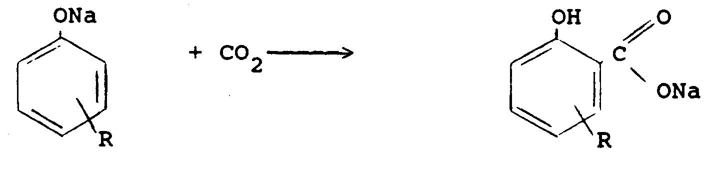
4. Furthermore the following equilibrium reaction occurs: 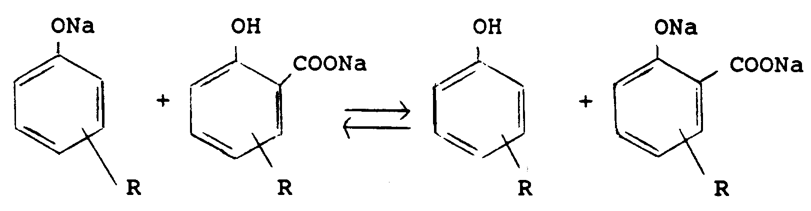 R is preferably an alkyl group having from 2 to 20 carbon atoms, more preferably from 10 to 18 carbon atoms.
   The alkylated phenol prepared during the alkylation reaction preferably contains a mixture of alkyl groups and may even contain up to 20% of dialkylated compounds, depending upon the reaction circumstances.
   When the equilibrium reaction mixture of 4 is purified by means of a membrane, the equilibrium reaction is shifted to the right, since the alkyl substituted phenol is withdrawn through osmosis.
   To prepare an overbased calcium alkylsalicylate the following reactions may take place.
5. acidification 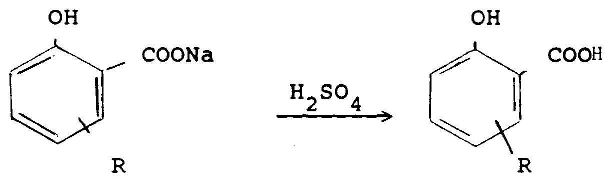
6. neutralization with Ca 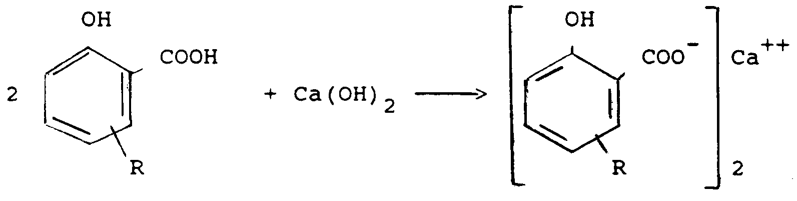 7. overbasing with Ca(OH)₂ + CO₂.

The phenation and carboxylation under 2 and 3 are preferably carried out in the presence of a solvent, such as an aromatic hydrocarbon. Examples of aromatic hydrocarbons are benzene, toluene and o-, m-, or p-xylene or mixtures thereof. Instead of using NaOH in the phenation reaction also KOH can be applied. The solvents especially the xylenes may serve as swelling agents for the used membranes.

Membranes are very well described in Kirk-Othmer "Encyclopedia of Chemical Technology" third edition, part 15, page 92 etc.

A very important and fundamental means by which a species can be transported through a membrane involves dissolving of the permeate molecules (alkylated phenols) into the membrane at its upstream surface, followed by molecular diffusion down its concentration gradient to the downstream face of the membrane. There the alkylated phenol is dissolved into its adjacent fluid phase.

The driving force for diffusion of the solvent through the membrane is the pressure exerted to the system. The driving force for phenol transport is the concentration gradient. In fact it is the pressure difference and the concentration difference on both sides of the membrane, which constitute the driving force.

Preferred membranes used in the process according to the invention are the dense cross-linked elastomeric membranes such as polyisoprene, polybutadiene, polydimethylsiloxane and fluorosilicon rubbers. Dense membranes generally have the ability to transport species selectively. Dense membranes may have, however, low transport rates. To attain acceptable transport rates, it is necessary to make the membranes thin, preferably very thin.

It may therefore be advantageous to bring the dense membranes on a porous support. The pressure exerted on the upstream face of the membrane is generally in the range of from 1 to 100 bar (100 to 10000 kPa) during the separation step. The temperature, used in the process according to the invention, ranges between 0 °C and 120 °C, preferably from 50 °C to 90 °C.

Of special importance to be used as membranes are the organopolysiloxanes, such as polydimethylsiloxane.

Only a part of the total flux through the membrane is alkylphenol. The permeate may be recirculated to the reactor or to the distillation column in the caustic reaction of the alkylphenol.

The membrane is highly selective for alkylphenol, since no sodium alkylsalicylate is found in the permeate.

The amount of alkylphenol in the carboxylated alkylphenol product (sodiumalkylsalicylate) can be considerably reduced. The alkylphenol can be used again in the carboxylation reaction. The xylene from the permeate, consisting of xylene and alkylphenol, can be distilled off and used either in the phenation reaction or as a swelling agent for the membrane separation process.

### EXAMPLE I

Caustic alkylphenol, containing C₁₄-C₁₈ alkyl groups, was admixed with xylene and the mixture was heated, so that an azeotropic mixture of xylene and water was distilled off. The azeotropic mixture was collected and separated in water and xylene. The xylene was recirculated to the caustic alkylphenol xylene mixture. The caustic alkylphenol was led into a reactor in which it was reacted with carbon dioxide in a xylene solution. The obtained reaction product comprising alkylphenol and sodium alkyl salicylate together with xylene was used in the experiment.

A solution of alkylphenol and sodium alkylsalicylate in xylene was pumped along the high pressure side of a polydimethylsiloxane membrane with an area of 10.2 cm², at a temperature of 60 °C and at permeation pressures between 5 and 40 bar (500 and 4000 kPa). The pressure was maintained via an air pressurized bellow and recirculation of the retentate solution was achieved via a rotary pump. The permeate solution was collected at the downstream side of the membrane out the recirculation system.

A feed consisting of 75.5% by weight of xylene and 24.5% by weight of alkylphenol and sodiumalkylsalicylate together (with an alkylphenol/sodiumsalicylate weight ratio of 0.21) gave after 6 h a retentate consisting of 66.4% by weight of xylene and 33.6% by weight of alkylphenol and sodiumalkylsalicylate together (with a weight ratio of alkylphenol/sodiumalkylsalicylate of 0.18). The permeate contained 94.5% by weight of xylene and 5.5% by weight of alkylphenol. No sodiumalkylsalicylate was found in the permeate.

The pressure, to which the membrane was subjected, was 5 bar during the first three hours, 20 bar at 3 h to 4 h and 40 bar the last two hours (at 4 h to 6 h) respectively. Every hour the flux was measured. The total flux through the polydimethylsiloxane membrane was 400 l/m².day, 1100 l/m².day and 1400 l/m².day at a pressure of 5, 20 and 40 bar respectively.

The alkylphenol flux through the polydimethylsiloxane membrane was at the beginning of the experiment 140 l/m².day, but decreased to 60 l/m².day at 5 bar pressure. Increasing the pressure to 20 bar and 40 bar led to an alkylphenol flux of 70 l/m².day and 80 l/m².day respectively. Consequently increase in pressure lead to a moderate increase in alkylphenol flux, but to a considerable increase in xylene flux. A highly selective separation of alkylphenol was obtained, since no sodiumalkylsalicylate could be detected in the permeate.

### EXAMPLE II

A feed consisting of 69.4% by weight of xylene and 30.6% by weight of alkylphenol and sodiumalkylsalicylate together, was pumped along the high pressure side of the polydimethylsiloxane membrane with an area of 10.2 cm² at a temperature of 60 °C and at a pressure of 20 bar (2000 kPa). The pressure was maintained via an air pressurized bellow and the recirculation of the retentate solution was achieved via a rotary pump. The permeate solution was collected at the downstream side of the membrane outside the recirculation system. The experiment lasted about 4 days. During the experiment the xylene concentration in the feed was held between 63 and 87% by weight of the feed by supplying fresh xylene to the feed at intervals, the reason for this measure being that xylene diffuses through the membrane at a high rate (compared with the alkylphenol diffusion rate).

In the following Table the results are given:

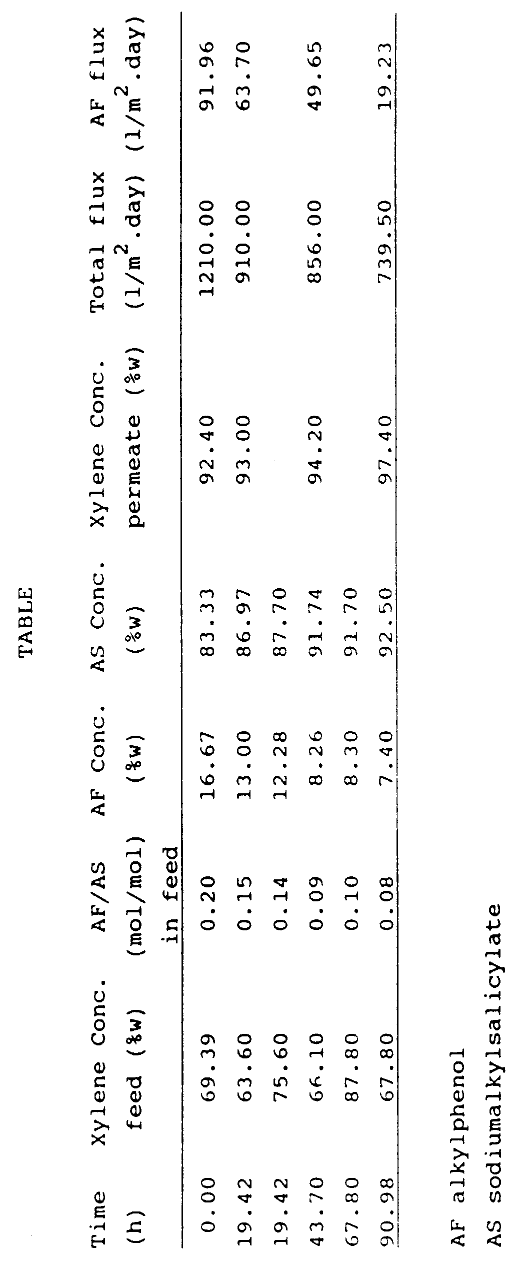

None of the permeates contained sodiumalkylsalicylic acid, consequently the polydimethylsiloxane membrane is highly selective to alkylphenol. After about 4 days the alkylphenol concentration in the original feed mixture is considerably reduced.

## Claims

1. A process for the separation of an alkylphenol, from a mixture obtained by the phenation and carboxylation of an alkylphenol having from 2 to 20 carbon atoms in the alkyl group, the mixture containing the alkylphenol and the corresponding alkylphenolate and alkylsalicylate, in an aromatic hydrocarbon solvent, characterized in that the separation is carried out by maintaining a positive pressure- and concentration gradient across a dense membrane which is selectively permeable to the alkylphenol and which swells by the aromatic hydrocarbon solvent.

2. A process according to claim 1, characterized in that the alkyl group has from 10 to 18 carbon atoms.

3. A process according to claim 1 or 2, characterized in that the alkyl phenolate is sodium- or potassium alkyl phenolate.

4. A process according to any one of claims 1-3, characterized in that the dense membrane is supported on a porous support.

5. A process according to any one of claims 1-4, characterized in that an organopolysiloxane is used as the dense membrane.

6. A process according to claim 5, characterised in that polydimethylsiloxane is used as the dense membrane.

7. A process according to any one of claims 1-6, characterized in that the aromatic hydrocarbon is a xylene.

8. A process according to any one of claims 1-7, characterized in that the positive pressure gradient is in the range of from 1 to 100 bar (100 to 10000 kPa).

9. A process according to any one of claims 1-8, characterized in that a temperature in the range of from 0 to 120 °C is applied.

10. A process according to claim 9, characterised in that the temperature is in the range of from 50 to 90 °C.

## Patentansprüche

1. Ein Verfahren zur Abtrennung von Alkylphenol aus einer Mischung, erhalten durch die Phenolatbildung und Carboxylierung eines Alkylphenols, welches zwischen 2 und 20 Kohlenstoffatome in der Alkylgruppe aufweist, wobei die Mischung das Alkylphenol und das entsprechende Alkylphenolat und Alkylsalicylat in einem aromatischen Kohlenwasserstofflösungsmittel enthält, dadurch gekennzeichnet, daß die Abtrennung durch Aufrechterhalten eines positiven Druck- und Konzentrationsgradienten quer durch eine dichte Membran, welche selektiv durchlässig für das Alkylphenol ist und welche durch das aromatische Kohlenwasserstofflösungsmittel aufquillt, durchgeführt wird.

2. Ein Verfahren gemäß Anspruch 1, dadurch gekennzeichnet, daß die Alkylgruppe zwischen 10 und 18 Kohlenstoffatome hat.

3. Ein Verfahren gemäß Anspruch 1 oder 2, dadurch gekennzeichnet, daß das Alkylphenolat Natrium- oder Kaliumalkylphenolat ist.

4. Ein Verfahren gemäß irgendeinem der Ansprüche 1-3, dadurch gekennzeichnet, daß die dichte Membran auf einen porösen Träger aufgebracht ist.

5. Ein Verfahren gemäß irgendeinem der Ansprüche 1-4, dadurch gekennzeichnet, daß ein Organopolysiloxan als dichte Membran verwendet wird.

6. Ein Verfahren gemäß Anspruch 5, dadurch gekennzeichnet, daß Polydimethylsiloxan als dichte Membran verwendet wird.

7. Ein Verfahren gemäß irgendeinem der Ansprüche 1-6, dadurch gekennzeichnet, daß der aromatische Kohlenwasserstoff ein Xylol ist.

8. Ein Verfahren gemäß irgendeinem der Ansprüche 1-7, dadurch gekennzeichnet, daß der positive Druckgradient im Bereich von 1 bis 100 bar liegt (100 bis 10000 kPa).

9. Ein Verfahren gemäß irgendeinem der Ansprüche 1-8, dadurch gekennzeichnet, daß eine Temperatur im Bereich von 0 bis 120 °C angewandt wird.

10. Ein Verfahren gemäß Anspruch 9, dadurch gekennzeichnet, daß die Temperatur im Bereich von 50 bis 90 °C liegt.

## Revendications

1. Un procédé de préparation d'un alcoylphénol à partir d'un mélange obtenu par la phénation et la carboxylation d'un alcoylphénol ayant de 2 à 20 atomes de carbone dans le groupe alcoyle, le mélange contenant l'alcoylphénol ainsi que l'alcoylphénolate et l'alcoylsalicylate correspondants dans un solvant hydrocarbure aromatique, caractérisé en ce que la séparation est effectuée en maintenant un gradient positif de pression et de concentration à travers une membrane dense qui est sélectivement perméable à l'alcoylphénol et qui est gonflée par le solvant hydrocarbure aromatique.

2. Un procédé selon la revendication 1, caractérisé en ce que le groupe alcoyle a de 10 à 18 atomes de carbone.

3. Un procédé selon la revendication 1 ou 2, caractérisé en ce que l'alcoylphénolate est un alcoylphénolate de sodium ou de potassium.

4. Un procédé selon l'une quelconque des revendications 1-3, caractérisé en ce que la membrane dense est supportée par un support poreux.

5. Un procédé selon l'une quelconque des revendications 1-4, caractérisé en ce qu'on utilise un organopolysiloxane comme membrane dense.

6. Un procédé selon la revendication 5, caractérisé en ce qu'on utilise du polydiméthylsiloxane comme membrane dense.

7. Un procédé selon l'une quelconque des revendications 1-6, caractérisé en ce que l'hydrocarbure aromatique est un xylène.

8. Un procédé selon l'une quelconque des revendications 1-7, caractérisé en ce que le gradient positif de pression est compris entre 1 et 100 bars (100 et 10 000 kPa).

9. Un procédé selon l'une quelconque des revendications 1-8, caractérisé en ce qu'on utilise une température comprise entre 0 et 120°C.

10. Un procédé selon la revendication 9, caractérisé en ce que la température est comprise entre 50 et 90°C.
